(19) Europäisches Patentamt
European Patent Office
Office européen des brevets

(11) EP 1 170 365 A1

(12) **EUROPEAN PATENT APPLICATION**

(43) Date of publication:
**09.01.2002 Bulletin 2002/02**

(21) Application number: **00202352.1**

(22) Date of filing: **04.07.2000**

(51) Int Cl.⁷: **C12N 15/12**, C07K 14/47, C07K 16/18, G01N 33/566, C12Q 1/68

(84) Designated Contracting States:
**AT BE CH CY DE DK ES FI FR GB GR IE IT LI LU MC NL PT SE**
Designated Extension States:
**AL LT LV MK RO SI**

(71) Applicant: **SMITHKLINE BEECHAM PLC**
**Brentford, Middlesex TW8 9EP (GB)**

(72) Inventors:
- **Smith, Graham,**
  **SmithKline Beecham Pharmaceuticals**
  **Harlow, Essex, CM19 5AW (GB)**
- **Hayes, Philip David,**
  **SmithKline Beecham Pharm.**
  **Harlow, Essex, CM19 5AW (GB)**
- **Smart, Darren,**
  **SmithKline Beecham Pharmaceuticals**
  **Harlow, Essex, CM19 5AW (GB)**
- **Davis, John Beresford,**
  **SmithKline Beecham Pharm.**
  **Harlow, Essex, CM19 5AW (GB)**
- **Kelsell, Rosemary Elizabeth,**
  **SmithKline Beecham P.**
  **Harlow, Essex, CM19 5AW (GB)**

(74) Representative: **Connell, Anthony Christopher**
**SmithKline Beecham plc Corporate Intellectual Property, Two New Horizons Court**
**Brentford, Middlesex TW8 9EP (GB)**

(54) **Member of the ion channel family of polypeptides; vanilrep4**

(57) VANILREP4 polypeptides and polynucleotides member of the ion channel family of polypeptides, and methods for producing such polypeptides by recombinant techniques are disclosed. Also disclosed are methods for utilizing VANILREP4 polypeptides and polynucleotides in diagnostic assays.

EP 1 170 365 A1

SEQUENCE LISTING

<110> SmithKline Beecham plc

<120> Novel Compounds

<130> GP30201

<160> 4

<170> FastSEQ for Windows Version 3.0

<210> 1
<211> 2616
<212> DNA
<213> Homo sapiens

<400> 1

```
atggcggatt ccagcgaagg cccccgcgcg gggcccgggg aggtggctga gctccccggg     60
gatgagagtg gcaccccagg tggggaggct tttcctctct cctccctggc caatctgttt    120
gagggggagg atggctccct ttcgccctca ccggctgatg ccagtcgccc tgctggccca    180
ggcgatgggc gaccaaatct gcgcatgaag ttccagggcg ccttccgcaa gggggtgccc    240
aaccccatcg atctgctgga gtccacccta tatgagtcct cggtggtgcc tgggcccaag    300
aaagcaccca tggactcact gtttgactac ggcacctatc gtcaccactc cagtgacaac    360
aagaggtgga ggaagaagat catagagaag cagccgcaga gccccaaagc ccctgcccct    420
cagccgcccc ccatcctcaa agtcttcaac cggcctatcc tctttgacat cgtgtcccgg    480
ggctccactg ctgacctgga cgggctgctc ccattcttgc tgacccacaa gaaacgccta    540
actgatgagg agtttcgaga gccatctacg ggaagacct gcctgcccaa ggccttgctg    600
aacctgagca atggccgcaa cgacaccatc cctgtgctgc tggacatcgc ggagcgcacc    660
ggcaacatgc gggagttcat taactcgccc ttccgtgaca tctactatcg aggtcagaca    720
gccctgcaca tcgccattga gcgtcgctgc aaacactacg tggaacttct cgtggcccag    780
ggagctgatg tccacgccca ggcccgtggg cgcttcttcc agcccaagga tgagggggc    840
tacttctact ttggggagct gcccctgtcg ctggctgcct gcaccaacca gccccacatt    900
gtcaactacc tgacggagaa cccccacaag aaggcggaca tgcggcgcca ggactcgcga    960
ggcaacacag tgctgcatgc gctggtggcc attgctgaca acacccgtga gaacaccaag   1020
tttgttacca agatgtacga cctgctgctg ctcaagtgtg cccgcctctt ccccgacagc   1080
aacctggagg ccgtgctcaa caacgacggc ctctcgcccc tcatgatggc tgccaagacg   1140
ggcaagattg ggatctttca gcacatcatc cggcgggagg tgacggatga ggacacacgg   1200
```

cacctgtccc gcaagttcaa ggactgggcc tatgggccag tgtattcctc gctttatgac 1260
ctctcctccc tggacacgtg tggggaagag gcctccgtgc tggagatcct ggtgtacaac 1320
agcaagattg agaaccgcca cgagatgctg gctgtggagc ccatcaatga actgctgcgg 1380
gacaagtggc gcaagttcgg ggccgtctcc ttctacatca acgtggtctc ctacctgtgt 1440
gccatggtca tcttcactct caccgcctac taccagccgc tggagggcac accgccgtac 1500
ccttaccgca ccacggtgga ctacctgcgg ctggctggcg aggtcattac gctcttcact 1560
ggggtcctgt tcttcttcac caacatcaaa gacttgttca tgaagaaatg ccctggagtg 1620
aattctctct tcattgatgg ctccttccag ctgctctact tcatctactc tgtcctggtg 1680
atcgtctcag cagccctcta cctggcaggg atcgaggcct acctggccgt gatggtcttt 1740
gccctggtcc tgggctggat gaatgccctt tacttcaccc gtgggctgaa gctgacgggg 1800
acctatagca tcatgatcca gaagattctc ttcaaggacc ttttccgatt cctgctcgtc 1860
tacttgctct tcatgatcgg ctacgcttca gccctggtct ccctcctgaa cccgtgtgcc 1920
aacatgaagg tgtgcaatga ggaccagacc aactgcacag tgcccactta cccctcgtgc 1980
cgtgacagcg agaccttcag caccttcctc ctggacctgt ttaagctgac cattggcatg 2040
ggcgacctgg agatgctgag cagcaccaag taccccgtgg tcttcatcat cctgctggtg 2100
acctacatca tcctcacctt tgtgctgctc ctcaacatgc tcattgccct catgggcgag 2160
acagtgggcc aggtctccaa ggagagcaag cacatctgga agctgcagtg ggccaccacc 2220
atcctggaca ttgagcgctc cttccccgta ttcctgagga aggccttccg ctctggggag 2280
atggtcaccg tgggcaagag ctcggacggc actcctgacc gcaggtggtg cttcagggtg 2340
gatgaggtga actggtctca ctggaaccag aacttgggca tcatcaacga ggacccgggc 2400
aagaatgaga cctaccagta ttatggcttc tcgcataccg tgggccgcct ccgcagggat 2460
cgctggtcct cggtggtacc ccgcgtggtg gaactgaaca agaactcgaa cccggacgag 2520
gtggtggtgc ctctggacag catggggaac cccgctgcg atggccacca gcagggttac 2580
ccccgcaagt ggaggactga tgacgccccg ctctag 2616

<210> 2
<211> 871
<212> PRT
<213> Homo sapiens

<400> 2
Met Ala Asp Ser Ser Glu Gly Pro Arg Ala Gly Pro Gly Glu Val Ala
1 5 10 15
Glu Leu Pro Gly Asp Glu Ser Gly Thr Pro Gly Gly Glu Ala Phe Pro
20 25 30
Leu Ser Ser Leu Ala Asn Leu Phe Glu Gly Glu Asp Gly Ser Leu Ser
35 40 45
Pro Ser Pro Ala Asp Ala Ser Arg Pro Ala Gly Pro Gly Asp Gly Arg
50 55 60
Pro Asn Leu Arg Met Lys Phe Gln Gly Ala Phe Arg Lys Gly Val Pro

```
65                  70                  75                  80
Asn Pro Ile Asp Leu Leu Glu Ser Thr Leu Tyr Glu Ser Ser Val Val
                85                  90                  95
Pro Gly Pro Lys Lys Ala Pro Met Asp Ser Leu Phe Asp Tyr Gly Thr
            100                 105                 110
Tyr Arg His His Ser Ser Asp Asn Lys Arg Trp Arg Lys Lys Ile Ile
        115                 120                 125
Glu Lys Gln Pro Gln Ser Pro Lys Ala Pro Ala Pro Gln Pro Pro Pro
    130                 135                 140
Ile Leu Lys Val Phe Asn Arg Pro Ile Leu Phe Asp Ile Val Ser Arg
145                 150                 155                 160
Gly Ser Thr Ala Asp Leu Asp Gly Leu Leu Pro Phe Leu Leu Thr His
                165                 170                 175
Lys Lys Arg Leu Thr Asp Glu Glu Phe Arg Glu Pro Ser Thr Gly Lys
            180                 185                 190
Thr Cys Leu Pro Lys Ala Leu Leu Asn Leu Ser Asn Gly Arg Asn Asp
        195                 200                 205
Thr Ile Pro Val Leu Leu Asp Ile Ala Glu Arg Thr Gly Asn Met Arg
    210                 215                 220
Glu Phe Ile Asn Ser Pro Phe Arg Asp Ile Tyr Tyr Arg Gly Gln Thr
225                 230                 235                 240
Ala Leu His Ile Ala Ile Glu Arg Arg Cys Lys His Tyr Val Glu Leu
                245                 250                 255
Leu Val Ala Gln Gly Ala Asp Val His Ala Gln Ala Arg Gly Arg Phe
            260                 265                 270
Phe Gln Pro Lys Asp Glu Gly Gly Tyr Phe Tyr Phe Gly Glu Leu Pro
        275                 280                 285
Leu Ser Leu Ala Ala Cys Thr Asn Gln Pro His Ile Val Asn Tyr Leu
    290                 295                 300
Thr Glu Asn Pro His Lys Lys Ala Asp Met Arg Arg Gln Asp Ser Arg
305                 310                 315                 320
Gly Asn Thr Val Leu His Ala Leu Val Ala Ile Ala Asp Asn Thr Arg
                325                 330                 335
Glu Asn Thr Lys Phe Val Thr Lys Met Tyr Asp Leu Leu Leu Leu Lys
            340                 345                 350
Cys Ala Arg Leu Phe Pro Asp Ser Asn Leu Glu Ala Val Leu Asn Asn
        355                 360                 365
Asp Gly Leu Ser Pro Leu Met Met Ala Ala Lys Thr Gly Lys Ile Gly
    370                 375                 380
Ile Phe Gln His Ile Ile Arg Arg Glu Val Thr Asp Glu Asp Thr Arg
```

385 390 395 400

His Leu Ser Arg Lys Phe Lys Asp Trp Ala Tyr Gly Pro Val Tyr Ser
405 410 415

Ser Leu Tyr Asp Leu Ser Ser Leu Asp Thr Cys Gly Glu Glu Ala Ser
420 425 430

Val Leu Glu Ile Leu Val Tyr Asn Ser Lys Ile Glu Asn Arg His Glu
435 440 445

Met Leu Ala Val Glu Pro Ile Asn Glu Leu Leu Arg Asp Lys Trp Arg
450 455 460

Lys Phe Gly Ala Val Ser Phe Tyr Ile Asn Val Val Ser Tyr Leu Cys
465 470 475 480

Ala Met Val Ile Phe Thr Leu Thr Ala Tyr Tyr Gln Pro Leu Glu Gly
485 490 495

Thr Pro Pro Tyr Pro Tyr Arg Thr Thr Val Asp Tyr Leu Arg Leu Ala
500 505 510

Gly Glu Val Ile Thr Leu Phe Thr Gly Val Leu Phe Phe Phe Thr Asn
515 520 525

Ile Lys Asp Leu Phe Met Lys Lys Cys Pro Gly Val Asn Ser Leu Phe
530 535 540

Ile Asp Gly Ser Phe Gln Leu Leu Tyr Phe Ile Tyr Ser Val Leu Val
545 550 555 560

Ile Val Ser Ala Ala Leu Tyr Leu Ala Gly Ile Glu Ala Tyr Leu Ala
565 570 575

Val Met Val Phe Ala Leu Val Leu Gly Trp Met Asn Ala Leu Tyr Phe
580 585 590

Thr Arg Gly Leu Lys Leu Thr Gly Thr Tyr Ser Ile Met Ile Gln Lys
595 600 605

Ile Leu Phe Lys Asp Leu Phe Arg Phe Leu Leu Val Tyr Leu Leu Phe
610 615 620

Met Ile Gly Tyr Ala Ser Ala Leu Val Ser Leu Leu Asn Pro Cys Ala
625 630 635 640

Asn Met Lys Val Cys Asn Glu Asp Gln Thr Asn Cys Thr Val Pro Thr
645 650 655

Tyr Pro Ser Cys Arg Asp Ser Glu Thr Phe Ser Thr Phe Leu Leu Asp
660 665 670

Leu Phe Lys Leu Thr Ile Gly Met Gly Asp Leu Glu Met Leu Ser Ser
675 680 685

Thr Lys Tyr Pro Val Val Phe Ile Ile Leu Leu Val Thr Tyr Ile Ile
690 695 700

Leu Thr Phe Val Leu Leu Leu Asn Met Leu Ile Ala Leu Met Gly Glu

705 710 715 720
Thr Val Gly Gln Val Ser Lys Glu Ser Lys His Ile Trp Lys Leu Gln
725 730 735
Trp Ala Thr Thr Ile Leu Asp Ile Glu Arg Ser Phe Pro Val Phe Leu
740 745 750
Arg Lys Ala Phe Arg Ser Gly Glu Met Val Thr Val Gly Lys Ser Ser
755 760 765
Asp Gly Thr Pro Asp Arg Arg Trp Cys Phe Arg Val Asp Glu Val Asn
770 775 780
Trp Ser His Trp Asn Gln Asn Leu Gly Ile Ile Asn Glu Asp Pro Gly
785 790 795 800
Lys Asn Glu Thr Tyr Gln Tyr Tyr Gly Phe Ser His Thr Val Gly Arg
805 810 815
Leu Arg Arg Asp Arg Trp Ser Ser Val Val Pro Arg Val Val Glu Leu
820 825 830
Asn Lys Asn Ser Asn Pro Asp Glu Val Val Val Pro Leu Asp Ser Met
835 840 845
Gly Asn Pro Arg Cys Asp Gly His Gln Gln Gly Tyr Pro Arg Lys Trp
850 855 860
Arg Thr Asp Asp Ala Pro Leu
865 870

<210> 3
<211> 3223
<212> DNA
<213> Homo sapiens

<400> 3
ccacgcgtcc gctcccggcc gccggcgccc agccgtcccg gaggctgagc agtgcagacg 60
ggcctggggc aggcatggcg gattccagcg aaggcccccg cgcggggccc ggggaggtgg 120
ctgagctccc cggggatgag agtggcaccc caggtgggga ggcttttcct ctctcctccc 180
tggccaatct gtttgagggg gaggatggct ccctttcgcc ctcaccggct gatgccagtc 240
gccctgctgg cccaggcgat gggcgaccaa atctgcgcat gaagttccag ggcgccttcc 300
gcaagggggt gcccaacccc atcgatctgc tggagtccac cctatatgag tcctcggtgg 360
tgcctgggcc caagaaagca cccatggact cactgtttga ctacggcacc tatcgtcacc 420
actccagtga caacaagagg tggaggaaga agatcataga gaagcagccg cagagcccca 480
aagcccctgc ccctcagccg ccccccatcc tcaaagtctt caaccggcct atcctctttg 540
acatcgtgtc ccggggctcc actgctgacc tggacgggct gctcccattc ttgctgaccc 600
acaagaaacg cctaactgat gaggagtttc gagagccatc tacggggaag acctgcctgc 660
ccaaggcctt gctgaacctg agcaatggcc gcaacgacac catccctgtg ctgctggaca 720

```
tcgcggagcg caccggcaac atgcgggagt tcattaactc gcccttccgt gacatctact     780
atcgaggtca gacagccctg cacatcgcca ttgagcgtcg ctgcaaacac tacgtggaac     840
ttctcgtggc ccagggagct gatgtccacg cccaggcccg tgggcgcttc ttccagccca     900
aggatgaggg gggctacttc tactttgggg agctgcccct gtcgctggct gcctgcacca     960
accagcccca cattgtcaac tacctgacgg agaaccccca caagaaggcg gacatgcggc    1020
gccaggactc gcgaggcaac acagtgctgc atgcgctggt ggccattgct gacaacaccc    1080
gtgagaacac caagtttgtt accaagatgt acgacctgct gctgctcaag tgtgcccgcc    1140
tcttccccga cagcaacctg gaggccgtgc tcaacaacga cggcctctcg cccctcatga    1200
tggctgccaa gacgggcaag attgggatct ttcagcacat catccggcgg gaggtgacgg    1260
atgaggacac acggcacctg tcccgcaagt tcaaggactg ggcctatggg ccagtgtatt    1320
cctcgcttta tgacctctcc tccctggaca cgtgtgggga agaggcctcc gtgctggaga    1380
tcctggtgta caacagcaag attgagaacc gccacgagat gctggctgtg gagcccatca    1440
atgaactgct gcgggacaag tggcgcaagt tcggggccgt ctccttctac atcaacgtgg    1500
tctcctacct gtgtgccatg gtcatcttca ctctcaccgc ctactaccag ccgctggagg    1560
gcacaccgcc gtacccttac cgcaccacgg tggactacct gcggctggct ggcgaggtca    1620
ttacgctctt cactggggtc ctgttcttct tcaccaacat caaagacttg ttcatgaaga    1680
aatgccctgg agtgaattct ctcttcattg atggctcctt ccagctgctc tacttcatct    1740
actctgtcct ggtgatcgtc tcagcagccc tctacctggc agggatcgag gcctacctgg    1800
ccgtgatggt ctttgccctg gtcctgggct ggatgaatgc cctttacttc acccgtgggc    1860
tgaagctgac ggggacctat agcatcatga tccagaagat tctcttcaag gacctttcc     1920
gattcctgct cgtctacttg ctcttcatga tcggctacgc ttcagccctg gtctccctcc    1980
tgaacccgtg tgccaacatg aaggtgtgca atgaggacca gaccaactgc acagtgccca    2040
cttacccctc gtgccgtgac agcgagacct tcagcacctt cctcctggac ctgtttaagc    2100
tgaccattgg catgggcgac ctggagatgc tgagcagcac caagtacccc gtggtcttca    2160
tcatcctgct ggtgacctac atcatcctca cctttgtgct gctcctcaac atgctcattg    2220
ccctcatggg cgagacagtg ggccaggtct ccaaggagag caagcacatc tggaagctgc    2280
agtgggccac caccatcctg gacattgagc gctccttccc cgtattcctg aggaaggcct    2340
tccgctctgg ggagatggtc accgtgggca agagctcgga cggcactcct gaccgcaggt    2400
ggtgcttcag ggtggatgag gtgaactggt ctcactggaa ccagaacttg ggcatcatca    2460
acgaggaccc gggcaagaat gagacctacc agtattatgg cttctcgcat accgtgggcc    2520
gcctccgcag ggatcgctgg tcctcggtgg taccccgcgt ggtggaactg aacaagaact    2580
cgaacccgga cgaggtggtg gtgcctctgg acagcatggg gaacccccgc tgcgatggcc    2640
accagcaggg ttacccccgc aagtggagga ctgatgacgc cccgctctag ggactgcagc    2700
ccagccccag cttctctgcc cactcatttc tagtccagcc gcatttcagc agtgccttct    2760
ggggtgtccc cccacaccct gctttggccc cagaggcgag ggaccagtgg aggtgccagg    2820
gaggccccag gaccctgtgg tcccctggct ctgcctcccc accctggggt gggggctccc    2880
ggccacctgt cttgctccta tggagtcaca taagccaacg ccagagcccc tccacctcag    2940
gccccagccc ctgcctctcc attatttatt tgctctgctc tcaggaagcg acgtgacccc    3000
tgccccagct ggaacctggc agaggcctta ggaccccgtt ccaagtgcac tgcccggcca    3060
agccccagcc tcagcctgcg cctgagctgc atgcgccacc atttttggca gcgtggcagc    3120
```

tttgcaaggg gctggggccc tcggcgtggg gccatgcctt ctgtgtgttc tgtagtgtct 3180
gggatttgcc ggtgctcaat aaatgtttat tcattgacgg tga 3223

<210> 4
<211> 3237
<212> DNA
<213> Homo sapiens

<400> 4
ccggccggga ttcaggaagc gcggatctcc cggccgccgg cgcccagccg tcccggaggc 60
tgagcagtgc agacgggcct ggggcaggca tggcggattc cagcgaaggc ccccgcgcgg 120
ggcccgggga ggtggctgag ctccccgggg atgagagtgg cacccccaggt ggggaggctt 180
ttcctctctc ctccctggcc aatctgtttg agggggagga tggctccctt tcgccctcac 240
cggctgatgc cagtcgccct gctggcccag gcgatgggcg accaaatctg cgcatgaagt 300
tccagggcgc cttccgcaag gggggtgccca accccatcga tctgctggag tccaccctat 360
atgagtcctc ggtggtgcct gggcccaaga aagcacccat ggactcactg tttgactacg 420
gcacctatcg tcaccactcc agtgacaaca agaggtggag gaagaagatc atagagaagc 480
agccgcagag ccccaaagcc cctgcccctc agccgccccc catcctcaaa gtcttcaacc 540
ggcctatcct ctttgacatc gtgtcccggg gctccactgc tgacctggac gggctgctcc 600
cattcttgct gacccacaag aaacgcctaa ctgatgagga gtttcgagag ccatctacgg 660
ggaagacctg cctgcccaag gccttgctga acctgagcaa tggccgcaac gacaccatcc 720
ctgtgctgct ggacatcgcg gagcgcaccg gcaacatgcg ggagttcatt aactcgccct 780
tccgtgacat ctactatcga ggtcagacag ccctgcacat cgccattgag cgtcgctgca 840
aacactacgt ggaacttctc gtggcccagg gagctgatgt ccacgcccag gcccgtgggc 900
gcttcttcca gcccaaggat gagggggggct acttctactt tggggagctg cccctgtcgc 960
tggctgcctg caccaaccag ccccacattg tcaactacct gacggagaac ccccacaaga 1020
aggcggacat gcggcgccag gactcgcgag gcaacacagt gctgcatgcg ctggtggcca 1080
ttgctgacaa cacccgtgag aacaccaagt ttgttaccaa gatgtacgac ctgctgctgc 1140
tcaagtgtgc ccgcctcttc cccgacagca acctggaggc cgtgctcaac aacgacggcc 1200
tctcgcccct catgatggct gccaagacgg gcaagattgg gatctttcag cacatcatcc 1260
ggcgggaggt gacggatgag gacacacggc acctgtcccg caagttcaag gactgggcct 1320
atgggccagt gtattcctcg ctttatgacc tctcctccct ggacacgtgt ggggaagagg 1380
cctccgtgct ggagatcctg gtgtacaaca gcaagattga gaaccgccac gagatgctgg 1440
ctgtggagcc catcaatgaa ctgctgcggg acaagtggcg caagttcggg gccgtctcct 1500
tctacatcaa cgtggtctcc tacctgtgtg ccatggtcat cttcactctc accgcctact 1560
accagccgct ggagggcaca ccgccgtacc cttaccgcac cacggtggac tacctgcggc 1620
tggctggcga ggtcattacg ctcttcactg gggtcctgtt cttcttcacc aacatcaaag 1680
acttgttcat gaagaaatgc cctggagtga attctctctt cattgatggc tccttccagc 1740
tgctctactt catctactct gtcctggtga tcgtctcagc agccctctac ctggcaggga 1800
tcgaggccta cctggccgtg atggtctttg ccctggtcct gggctggatg aatgcccttt 1860

```
acttcacccg tgggctgaag ctgacgggga cctatagcat catgatccag aagattctct    1920
tcaaggacct tttccgattc ctgctcgtct acttgctctt catgatcggc tacgcttcag    1980
ccctggtctc cctcctgaac ccgtgtgcca acatgaaggt gtgcaatgag gaccagacca    2040
actgcacagt gcccacttac ccctcgtgcc gtgacagcga gaccttcagc accttcctcc    2100
tggacctgtt taagctgacc attggcatgg gcgacctgga gatgctgagc agcaccaagt    2160
accccgtggt cttcatcatc ctgctggtga cctacatcat cctcaccttt gtgctgctcc    2220
tcaacatgct cattgccctc atgggcgaga cagtgggcca ggtctccaag gagagcaagc    2280
acatctggaa gctgcagtgg gccaccacca tcctggacat tgagcgctcc ttccccgtat    2340
tcctgaggaa ggccttccgc tctggggaga tggtcaccgt gggcaagagc tcggacggca    2400
ctcctgaccg caggtggtgc ttcagggtgg atgaggtgaa ctggtctcac tggaaccaga    2460
acttgggcat catcaacgag gacccgggca agaatgagac ctaccagtat tatggcttct    2520
cgcataccgt gggccgcctc cgcagggatc gctggtcctc ggtggtaccc cgcgtggtgg    2580
aactgaacaa gaactcgaac ccggacgagg tggtggtgcc tctggacagc atggggaacc    2640
cccgctgcga tggccaccag cagggttacc cccgcaagtg gaggactgat gacgccccgc    2700
tctagggact gcagcccagc cccagcttct ctgcccactc atttctagtc cagccgcatt    2760
tcagcagtgc cttctggggt gtcccccac accctgcttt ggccccagag gcgagggacc    2820
agtggaggtg ccagggaggc cccaggaccc tgtggtcccc tggctctgcc tccccaccct    2880
ggggtggggg ctcccggcca cctgtcttgc tcctatggag tcacataagc caacgccaga    2940
gcccctccac ctcaggcccc agcccctgcc tctccattat ttatttgctc tgctctcagg    3000
aagcgacgtg acccctgccc cagctggaac ctggcagagg ccttaggacc ccgttccaag    3060
tgcactgccc ggccaagccc cagcctcagc ctgcgcctga gctgcatgcg ccaccatttt    3120
tggcagcgtg gcagctttgc aaggggctgg ggccctcggc gtggggccat gccttctgtg    3180
tgttctgtag tgtctgggat ttgccggtgc tcaataaatg tttattcatt gacggtg       3237
```

## Description

### Field of the Invention

This invention relates to newly identified polypeptides and polynucleotides encoding such polypeptides, to their use in diagnosis and in identifying compounds that may be agonists, antagonists that are potentially useful in therapy, and to production of such polypeptides and polynucleotides.

### Background of the Invention

The drug discovery process is currently undergoing a fundamental revolution as it embraces "functional genomics", that is, high throughput genome- or gene-based biology. This approach as a means to identify genes and gene products as therapeutic targets is rapidly superceding earlier approaches based on "positional cloning". A phenotype, that is a biological function or genetic disease, would be identified and this would then be tracked back to the responsible gene, based on its genetic map position.

Functional genomics relies heavily on high-throughput DNA sequencing technologies and the various tools of bioinformatics to identify gene sequences of potential interest from the many molecular biology databases now available. There is a continuing need to identify and characterise further genes and their related polypeptides/proteins, as targets for drug discovery.

### Summary of the Invention

The present invention relates to VANILREP4, in particular VANILREP4 polypeptides and VANILREP4 polynucleotides, recombinant materials and methods for their production. Such polypeptides and polynucleotides are of interest in relation to methods of treatment of certain diseases, including, but not limited to, pain, chronic pain, neuropathic pain, postoperative pain, rheumatoid arthritic pain, neuralgia, neuropathies, algesia, nerve injury, ischaemia, neurodegeneration, stroke, incontinence, inflammatory disorders, irritable bowel syndrome, diabetes or obesity, hereinafter referred to as " diseases of the invention". In a further aspect, the invention relates to methods for identifying agonists and antagonists (e.g., inhibitors) using the materials provided by the invention, and treating conditions associated with VANILREP4 imbalance with the identified compounds. In a still further aspect, the invention relates to diagnostic assays for detecting diseases associated with inappropriate VANILREP4 activity or levels.

### Description of the Invention

In a first aspect, the present invention relates to VANILREP4 polypeptides. Such polypeptides include:

(a) an isolated polypeptide encoded by a polynucleotide comprising the sequence of SEQ ID NO:1;
(b) an isolated polypeptide comprising a polypeptide sequence having at least 95%, 96%, 97%, 98%, or 99% identity to the polypeptide sequence of SEQ ID NO:2;
(c) an isolated polypeptide comprising the polypeptide sequence of SEQ ID NO:2;
(d) an isolated polypeptide having at least 95%, 96%, 97%, 98%, or 99% identity to the polypeptide sequence of SEQ ID NO:2;
(e) the polypeptide sequence of SEQ ID NO:2; and
(f) an isolated polypeptide having or comprising a polypeptide sequence that has an Identity Index of 0.95, 0.96, 0.97, 0.98, or 0.99 compared to the polypeptide sequence of SEQ ID NO:2;
(g) fragments and variants of such polypeptides in (a) to (f).

Polypeptides of the present invention are believed to be members of the ion channel family of polypeptides. They are therefore of interest because they are related to the VR1 channel which is associated with the mechanism of action of capsaicin (a vanilloid compound), a constituent of chilli peppers. Capsaicin elicits a sensation of burning pain by selectively activating sensory neurons that convey information about noxious stimuli to the central nervous system. The channels are permeable to cations and exhibit a notable preferance for divalent cations, particularly calcium ions. The level of calcium ion permeability exceeds that observed for most non-selective cation channels and is similar to values observed for NMDA-type glutamate receptors and alpha? nicotinic acetylcholine receptors, both of which are noted for this property. Ion channels are particularly important in homeostasis and signalling pathways, thus being attractive targets for therapeutic intervention..

The biological properties of the VANILREP4 are hereinafter referred to as "biological activity of VANILREP4" or VANILREP4 activity". Preferably, a polypeptide of the present invention exhibits at least one biological activity of VANILREP4. Polypeptides of the present invention also includes variants of the aforementioned polypeptides, including all allelic forms and splice variants. Such polypeptides vary from the reference polypeptide by insertions, deletions, and substitutions that may be conservative or non-conservative, or any combination thereof. Particularly preferred variants are those in which several, for instance from 50 to 30, from 30 to 20, from 20 to 10, from 10 to 5, from 5 to 3, from 3 to 2, from 2 to 1 or 1 amino acids are inserted, substituted, or deleted, in any combination.

Preferred fragments of polypeptides of the present invention include an isolated polypeptide comprising an amino acid sequence having at least 30, 50 or 100 contiguous amino acids from the amino acid sequence of SEQ ID NO: 2, or an isolated polypeptide comprising an amino acid sequence having at least 30, 50 or 100 contiguous amino acids truncated or deleted from the amino acid sequence of SEQ ID NO: 2. Preferred fragments are biologically active fragments that mediate the biological activity of VANILREP4, including those with a similar activity or an improved activity, or with a decreased undesirable activity. Also preferred are those fragments that are antigenic or immunogenic in an animal, especially in a human.

Fragments of the polypeptides of the invention may be employed for producing the corresponding full-length polypeptide by peptide synthesis; therefore, these variants may be employed as intermediates for producing the full-length polypeptides of the invention.The polypeptides of the present invention may be in the form of the "mature" protein or may be a part of a larger protein such as a precursor or a fusion protein. It is often advantageous to include an additional amino acid sequence that contains secretory or leader sequences, pro-sequences, sequences that aid in purification, for instance multiple histidine residues, or an additional sequence for stability during recombinant production.

Polypeptides of the present invention can be prepared in any suitable manner, for instance by isolation form naturally occuring sources, from genetically engineered host cells comprising expression systems *(vide infra*) or by chemical synthesis, using for instance automated peptide synthesisers, or a combination of such methods. Means for preparing such polypeptides are well understood in the art.

In a further aspect, the present invention relates to VANILREP4 polynucleotides. Such polynucleotides include:

(a) an isolated polynucleotide comprising a polynucleotide sequence having at least 95%, 96%, 97%, 98%, or 99% identity to the polynucleotide squence of SEQ ID NO:1;
(b) an isolated polynucleotide comprising the polynucleotide of SEQ ID NO:1;
(c) an isolated polynucleotide having at least 95%, 96%, 97%, 98%, or 99% identity to the polynucleotide of SEQ ID NO:1;
(d) the isolated polynucleotide of SEQ ID NO:1;
(e) an isolated polynucleotide comprising a polynucleotide sequence encoding a polypeptide sequence having at least 95%, 96%, 97%, 98%, or 99% identity to the polypeptide sequence of SEQ ID NO:2;
(f) an isolated polynucleotide comprising a polynucleotide sequence encoding the polypeptide of SEQ ID NO:2;
(g) an isolated polynucleotide having a polynucleotide sequence encoding a polypeptide sequence having at least 95%, 96%, 97%, 98%, or 99% identity to the polypeptide sequence of SEQ ID NO:2;
(h) an isolated polynucleotide encoding the polypeptide of SEQ ID NO:2;
(i) an isolated polynucleotide having or comprising a polynucleotide sequence that has an Identity Index of 0.95, 0.96, 0.97, 0.98, or 0.99 compared to the polynucleotide sequence of SEQ ID NO:1;
(j) an isolated polynucleotide having or comprising a polynucleotide sequence encoding a polypeptide sequence that has an Identity Index of 0.95, 0.96, 0.97, 0.98, or 0.99 compared to the polypeptide sequence of SEQ ID NO: 2; and

polynucleotides that are fragments and variants of the above mentioned polynucleotides or that are complementary to above mentioned polynucleotides, over the entire length thereof.

Preferred fragments of polynucleotides of the present invention include an isolated polynucleotide comprising an nucleotide sequence having at least 15, 30, 50 or 100 contiguous nucleotides from the sequence of SEQ ID NO: 1, or an isolated polynucleotide comprising an sequence having at least 30, 50 or 100 contiguous nucleotides truncated or deleted from the sequence of SEQ ID NO: 1.

Preferred variants of polynucleotides of the present invention include splice variants, allelic variants, and polymorphisms, including polynucleotides having one or more single nucleotide polymorphisms (SNPs).

Polynucleotides of the present invention also include polynucleotides encoding polypeptide variants that comprise the amino acid sequence of SEQ ID NO:2 and in which several, for instance from 50 to 30, from 30 to 20, from 20 to 10, from 10 to 5, from 5 to 3, from 3 to 2, from 2 to 1 or 1 amino acid residues are substituted, deleted or added, in any combination.

In a further aspect, the present invention provides polynucleotides that are RNA transcripts of the DNA sequences of the present invention. Accordingly, there is provided an RNA polynucleotide that:

(a) comprises an RNA transcript of the DNA sequence encoding the polypeptide of SEQ ID NO:2;
(b) is the RNA transcript of the DNA sequence encoding the polypeptide of SEQ ID NO:2;
(c) comprises an RNA transcript of the DNA sequence of SEQ ID NO:1; or
(d) is the RNA transcript of the DNA sequence of SEQ ID NO:1;

and RNA polynucleotides that are complementary thereto.

The polynucleotide sequence of SEQ ID NO:1 shows homology with rat vanilloid receptor, VR1 (M. J. Caterina et al., Nature 389: 816-824, 1997). The polynucleotide sequence of SEQ ID NO:1 is a cDNA sequence that encodes the polypeptide of SEQ ID NO:2. The polynucleotide sequence encoding the polypeptide of SEQ ID NO:2 may be identical to the polypeptide encoding sequence of SEQ ID NO:1 or it may be a sequence other than SEQ ID NO:1, which, as a result of the redundancy (degeneracy) of the genetic code, also encodes the polypeptide of SEQ ID NO: 2. The polypeptide of the SEQ ID NO:2 is related to other proteins of the ion channel family, having homology and/or structural similarity with with rat vanilloid receptor, VR1 (M. J. Caterina et al., Nature 389: 816-824, 1997). The nucleotide sequence of SEQ ID NO:4 is a cDNA sequence and comprises a polypeptide encoding sequence (nucleotide 90 to 2705, Exonl (< 1-58), Exon 2 (59-475), Exon 3 (476-648), Exon 4 (649-801), Exon 5 (802-942), Exon 6 (943-1241), Exon 7 (1242-1421), Exon 8 (1422-1580), Exon 9 (1581-1673), Exon 10 (1674-1747), Exon 11 (1748-1913), Exon 12 (1914-1980), Exon 13 (1981-2297), Exon 14 (2298-2425), Exon 15 (2426-2546), Exon 16 (2547 - >3237)) encoding a polypeptide of 871 amino acids, the polypeptide of SEQ ID NO:2. Knowledge of the intron-exon structure of VANILREP4 can be used for mutation screening, for example as a diagnostic test for diseases which may be caused by alterations of VANILREP4. The screening of genomic DNA is desirable for the analysis of non-coding regions, such as upstream regulatory regions and intron splice sites. It is also useful in cases where mRNA is not readily available for mutation analysis. Knowledge of the genomic structure is also important for the generation of animal models. Such models may be used to study the function of VANILREP4 and for drug screening studies. For example,mouse knock-out models typically have a selection marker, which upon insertion into a coding exon, ablate the functioning of the targeted allele. The genomic structure may also be useful in analysing possible splice variants of VANILREP4. Splice variants are important because they may have different functions and different expression patterns.

Preferred polypeptides and polynucleotides of the present invention are expected to have, *inter alia*, similar biological functions/properties to their homologous polypeptides and polynucleotides. Furthermore, preferred polypeptides and polynucleotides of the present invention have at least one VANILREP4 activity.

The present invention also relates to partial or other polynucleotide and polypeptide sequences which were first identified prior to the determination of the corresponding full length sequence of SEQ ID NO4Accordingly, in a further aspect, the present invention provides for an isolated polynucleotide which:

(a) comprises a nucleotide sequence which has at least 95% identity, preferably at least 97-99% identity to SEQ ID NO:3 over the entire length of SEQ ID NO:3;
(b) has a nucleotide sequence which has at least 95% identity, preferably at least 97-99% identity, to SEQ ID NO: 3 over the entire length of SEQ ID NO:3; or
(c) the polynucleotide of SEQ ID NO:3.

The nucleotide sequence of SEQ ID NO:3 is derived from a single cDNA clone. The 5' 11bp of SEQ ID NO: 3 are at variance with the equivalent region of SEQ ID NO:4, and most likely result from nucleotide sequence reading errors of this first cDNA clone, or are the result of a cloning artefact. It is well known in the art that cDNA cloning and sequencing can be vulnerable to such problems (see Aaronson, J.S. et al. Genome Research 6:829-845 1996). The entire coding regions of the polynucleotides of SEQ ID NO:1, SEQ ID NO:3 and SEQ ID NO:4 are identical, and hence the polypeptides encoded therefrom are also identical, having the amino acid sequence of SEQ ID NO:2.

Polynucleotides of the present invention may be obtained using standard cloning and screening techniques from a cDNA library derived from mRNA in cells of human brain, germ cell, whole embryo, heart, kidney, prostate, lung, uterus, glioblastoma, adneocarcinoma, senescent fibroblast and osteoarthritic cartilage, (see for instance, Sambrook *et al.*, Molecular Cloning: A Laboratory Manual, 2nd Ed., Cold Spring Harbor Laboratory Press, Cold Spring Harbor, N.Y. (1989)). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself, or the coding sequence for the mature polypeptide in reading frame with other coding sequences, such as those encoding a leader or secretory sequence, a pre-, or pro- or prepro- protein sequence, or other fusion peptide portions. For example, a marker sequence that facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al.*, Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Polynucleotides that are identical, or have sufficient identity to a polynucleotide sequence of SEQ ID NO: 1, may be used as hybridization probes for cDNA and genomic DNA or as primers for a nucleic acid amplification reaction (for instance, PCR). Such probes and primers may be used to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1, typically at least 95% identity. Preferred probes and primers will generally comprise at least 15 nucleotides, preferably, at least 30 nucleotides and may have at least 50, if not at least 100 nucleotides. Particularly preferred probes will have between 30 and 50 nucleotides. Particularly preferred primers will have between 20 and 25 nucleotides.

A polynucleotide encoding a polypeptide of the present invention, including homologs from species other than human, may be obtained by a process comprising the steps of screening a library under stringent hybridization conditions with a labeled probe having the sequence of SEQ ID NO: 1 or a fragment thereof, preferably of at least 15 nucleotides; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA; followed by washing the filters in 0.1x SSC at about 65°C. Thus the present invention also includes isolated polynucleotides, preferably with a nucleotide sequence of at least 100, obtained by screening a library under stringent hybridization conditions with a labeled probe having the sequence of SEQ ID NO:1 or a fragment thereof, preferably of at least 15 nucleotides.

The skilled artisan will appreciate that, in many cases, an isolated cDNA sequence will be incomplete, in that the region coding for the polypeptide does not extend all the way through to the 5' terminus. This is a consequence of reverse transcriptase, an enzyme with inherently low "processivity" (a measure of the ability of the enzyme to remain attached to the template during the polymerisation reaction), failing to complete a DNA copy of the mRNA template during first strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., Proc Nat Acad Sci USA 85, 8998-9002, 1988). Recent modifications of the technique, exemplified by the Marathon (trade mark) technology (Clontech Laboratories Inc.) for example, have significantly simplified the search for longer cDNAs. In the Marathon (trade mark) technology, cDNAs have been prepared from mRNA extracted from a chosen tissue and an 'adaptor' sequence ligated onto each end. Nucleic acid amplification (PCR) is then carried out to amplify the "missing" 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, in a further aspect, the present invention relates to expression systems comprising a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Polynucleotides may be introduced into host cells by methods described in many standard laboratory manuals, such as Davis et al., Basic Methods in Molecular Biology (1986) and Sambrook *et al.*(*ibid*). Preferred methods of introducing polynucleotides into host cells include, for instance, calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *Streptococci*, *Staphylococci*, *E. coli*, *Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, *e.g.*, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector that is able to maintain, propagate or express a polynucleotide to produce a polypeptide in a host may be used. The appropriate polynucleotide sequence may be inserted into an expression system by any of a variety of well-known and routine techniques, such as, for example, those set forth in Sambrook *et al*., (*ibid*). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and/or purification.

Polynucleotides of the present invention may be used as diagnostic reagents, through detecting mutations in the associated gene. Detection of a mutated form of the gene characterised by the polynucleotide of SEQ ID NO: I in the cDNA or genomic sequence and which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques well known in the art.

Nucleic acids for diagnosis may be obtained from a subject's cells, such as from blood, urine, saliva, tissue biopsy or autopsy material. The genomic DNA may be used directly for detection or it may be amplified enzymatically by using PCR, preferably RT-PCR, or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled VANILREP4 nucleotide sequences. Perfectly matched sequences can be distinguished from mismatched duplexes by RNase digestion or by differences in melting temperatures. DNA sequence difference may also be detected by alterations in the electrophoretic mobility of DNA fragments in gels, with or without denaturing agents, or by direct DNA sequencing (see, for instance, Myers *et al*., Science (1985) 230:1242). Sequence changes at specific locations may also be revealed by nuclease protection assays, such as RNase and S protection or the chemical cleavage method (see Cotton *et al*., Proc Natl Acad Sci USA (1985) 85: 4397-4401).

An array of oligonucleotides probes comprising VANILREP4 polynucleotide sequence or fragments thereof can be constructed to conduct efficient screening of *e.g.,* genetic mutations. Such arrays are preferably high density arrays or grids. Array technology methods are well known and have general applicability and can be used to address a variety of questions in molecular genetics including gene expression, genetic linkage, and genetic variability, see, for example, M.Chee et al., Science, 274, 610-613 (1996) and other references cited therein.

Detection of abnormally decreased or increased levels of polypeptide or mRNA expression may also be used for diagnosing or determining susceptibility of a subject to a disease of the invention. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention relates to a diagonostic kit comprising:

(a) a polynucleotide of the present invention, preferably the nucleotide sequence of SEQ ID NO: 1, or a fragment or an RNA transcript thereof;
(b) a nucleotide sequence complementary to that of (a);
(c) a polypeptide of the present invention, preferably the polypeptide of SEQ ID NO:2 or a fragment thereof; or
(d) an antibody to a polypeptide of the present invention, preferably to the polypeptide of SEQ ID NO:2.

It will be appreciated that in any such kit, (a), (b), (c) or (d) may comprise a substantial component. Such a kit will be of use in diagnosing a disease or susceptibility to a disease, particularly diseases of the invention, amongst others.

The polynucleotide sequences of the present invention are valuable for chromosome localisation studies. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (co-inheritance of physically adjacent genes). Precise human chromosomal localisations for a genomic sequence (gene fragment etc.) can be determined using Radiation Hybrid (RH) Mapping (Walter, M. Spillett, D., Thomas, P., Weissenbach, J., and Goodfellow, P., (1994) A method for constructing radiation hybrid maps of whole genomes, Nature Genetics 7, 22-28). A number of RH panels are available from Research Genetics (Huntsville, AL, USA) e.g. the GeneBridge4 RH panel (Hum Mol Genet 1996 Mar;5(3):339-46 A radiation hybrid map of the human genome. Gyapay G, Schmitt K, Fizames C, Jones H, Vega-Czarny N, Spillett D, Muselet D, Prud'Homme JF, Dib C, Auffray C, Morissette J, Weissenbach J, Goodfellow PN). To determine the chromosomal location of a gene using this panel, 93 PCRs are performed using primers designed from the gene of interest on RH DNAs. Each of these DNAs contains random human genomic fragments maintained in a hamster background (human / hamster hybrid cell lines). These PCRs result in 93 scores indicating the presence or absence of the PCR product of the gene of interest. These scores are compared with scores created using PCR products from genomic sequences of known location. This comparison is conducted at http://www.genome.wi.mit.edu/. The gene of the present invention maps to human chromosome 12q24.1.

The polynucleotide sequences of the present invention are also valuable tools for tissue expression studies. Such studies allow the determination of expression patterns of polynucleotides of the present invention which may give an indication as to the expression patterns of the encoded polypeptides in tissues, by detecting the mRNAs that encode them. The techniques used are well known in the art and include in situ hydridisation techniques to clones arrayed on a grid, such as cDNA microarray hybridisation (Schena *et al*, Science, 270, 467-470, 1995 and Shalon *et al*, Genome Res, 6, 639-645, 1996) and nucleotide amplification techniques such as PCR. A preferred method uses the TAQMAN (Trade mark) technology available from Perkin Elmer. Results from these studies can provide an indication of the normal function of the polypeptide in the organism. In addition, comparative studies of the normal expression pattern of mRNAs with that of mRNAs encoded by an alternative form of the same gene (for example, one having an alteration in polypeptide coding potential or a regulatory mutation) can provide valuable insights into the role of the polypeptides of the present invention, or that of inappropriate expression thereof in disease. Such inappropriate expression may be of a temporal, spatial or simply quantitative nature.

The polynucleotides of the present invention are expressed in several tissues, including human brain, germ cell, whole embryo, heart, kidney, prostate, lung, uterus, glioblastoma, adneocarcinoma, senescent fibroblast ,osteoarthritic cartilage and sensory ganglia.

A further aspect of the present invention relates to antibodies. The polypeptides of the invention or their fragments, or cells expressing them, can be used as immunogens to produce antibodies that are immunospecific for polypeptides of the present invention. The term "immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256: 495-497), the trioma technique, the human B-cell hybridoma technique (Kozbor *et al.*, Immunology Today (1983) 4: 72) and the EBV-hybridoma technique (Cole *et al.*, Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography. Antibodies against polypeptides of the present invention may also be employed to treat diseases of the invention, amongst others.

Polypeptides and polynucleotides of the present invention may also be used as vaccines. Accordingly, in a further aspect, the present invention relates to a method for inducing an immunological response in a mammal that comprises inoculating the mammal with a polypeptide of the present invention, adequate to produce antibody and/or T cell immune response, including, for example, cytokine-producing T cells or cytotoxic T cells, to protect said animal from disease, whether that disease is already established within the individual or not. An immunological response in a mammal may also be induced by a method comprises delivering a polypeptide of the present invention *via* a vector directing expression of the polynucleotide and coding for the polypeptide *in vivo* in order to induce such an immunological response to produce antibody to protect said animal from diseases of the invention. One way of administering the vector is by accelerating it into the desired cells as a coating on particles or otherwise. Such nucleic acid vector may comprise DNA, RNA, a modified nucleic acid, or a DNA/RNA hybrid. For use a vaccine, a polypeptide or a nucleic acid vector will be normally provided as a vaccine formulation (composition). The formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions that may contain anti-oxidants, buffers, bacteriostats and solutes that render the formulation instonic with the blood of the recipient; and aqueous and non-aqueous sterile suspensions that may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention have one or more biological functions that are of relevance in one or more disease states, in particular the diseases of the invention hereinbefore mentioned. It is therefore useful to to identify compounds that stimulate or inhibit the function or level of the polypeptide. Accordingly, in a further aspect, the present invention provides for a method of screening compounds to identify those that stimulate or inhibit the function or level of the polypeptide. Such methods identify agonists or antagonists that may be employed for therapeutic and prophylactic purposes for such diseases of the invention as hereinbefore mentioned. Compounds may be identified from a variety of sources, for example, cells, cell-free preparations, chemical libraries, collections of chemical compounds, and natural product mixtures. Such agonists or antagonists so-identified may be natural or modified substrates, ligands, receptors, enzymes, etc., as the case may be, of the polypeptide; a structural or functional mimetic thereof (see Coligan *et al.*, Current Protocols in Immunology 1(2):Chapter 5 (1991)) or a small molecule. Such small molecules preferably have a molecular weight below 2,000 daltons, more preferably between 300 and 1,000 daltons, and most preferably between 400 and 700 daltons. It is preferred that these small molecules are organic molecules.

The screening method may simply measure the binding of a candidate compound to the polypeptide, or to cells or membranes bearing the polypeptide, or a fusion protein thereof, by means of a label directly or indirectly associated with the candidate compound. Alternatively, the screening method may involve measuring or detecting (qualitatively or quantitatively) the competitive binding of a candidate compound to the polypeptide against a labeled competitor *(e.g.* agonist or antagonist). Further, these screening methods may test whether the candidate compound results in a signal generated by activation or inhibition of the polypeptide, using detection systems appropriate to the cells bearing the polypeptide. Inhibitors of activation are generally assayed in the presence of a known agonist and the effect on activation by the agonist by the presence of the candidate compound is observed. Further, the screening methods may simply comprise the steps of mixing a candidate compound with a solution containing a polypeptide of the present invention, to form a mixture, measuring a VANILREP4 activity in the mixture, and comparing the VANILREP4 activity of the mixture to a control mixture which contains no candidate compound.

Polypeptides of the present invention may be employed in conventional low capacity screening methods and also in high-throughput screening (HTS) formats. Such HTS formats include not only the well-established use of 96- and, more recently, 384-well micotiter plates but also emerging methods such as the nanowell method described by Schullek et al, Anal Biochem., 246, 20-29, (1997).

Fusion proteins, such as those made from Fc portion and VANILREP4 polypeptide, as hereinbefore described, can also be used for high-throughput screening assays to identify antagonists for the polypeptide of the present invention (see D. Bennett *et al*., J Mol Recognition, 8:52-58 (1995); and K. Johanson *et al*., J Biol Chem, 270(16):9459-9471 (1995)).

The polynucleotides, polypeptides and antibodies to the polypeptide of the present invention may also be used to configure screening methods for detecting the effect of added compounds on the production of mRNA and polypeptide in cells. For example, an ELISA assay may be constructed for measuring secreted or cell associated levels of polypeptide using monoclonal and polyclonal antibodies by standard methods known in the art. This can be used to discover agents that may inhibit or enhance the production of polypeptide (also called antagonist or agonist, respectively) from suitably manipulated cells or tissues.

A polypeptide of the present invention may be used to identify membrane bound or soluble receptors, if any, through standard receptor binding techniques known in the art. These include, but are not limited to, ligand binding and crosslinking assays in which the polypeptide is labeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide that compete with the binding of the polypeptide to its receptors, if any. Standard methods for conducting such assays are well understood in the art.

Examples of antagonists of polypeptides of the present invention include antibodies or, in some cases, oligonucleotides or proteins that are closely related to the ligands, substrates, receptors, enzymes, etc., as the case may be, of the polypeptide, *e.g.*, a fragment of the ligands, substrates, receptors, enzymes, etc.; or a small molecule that bind to the polypeptide of the present invention but do not elicit a response, so that the activity of the polypeptide is prevented.

Screening methods may also involve the use of transgenic technology and VANILREP4 gene. The art of constructing transgenic animals is well established. For example, the VANILREP4 gene may be introduced through microinjection into the male pronucleus of fertilized oocytes, retroviral transfer into pre- or post-implantation embryos, or injection of genetically modified, such as by electroporation, embryonic stem cells into host blastocysts. Particularly useful transgenic animals are so-called "knock-in" animals in which an animal gene is replaced by the human equivalent within the genome of that animal. Knock-in transgenic animals are useful in the drug discovery process, for target validation, where the compound is specific for the human target. Other useful transgenic animals are so-called "knock-out" animals in which the expression of the animal ortholog of a polypeptide of the present invention and encoded by an endogenous DNA sequence in a cell is partially or completely annulled. The gene knock-out may be targeted to specific cells or tissues, may occur only in certain cells or tissues as a consequence of the limitations of the technology, or may occur in all, or substantially all, cells in the animal. Transgenic animal technology also offers a whole animal expression-cloning system in which introduced genes are expressed to give large amounts of polypeptides of the present invention

Screening kits for use in the above described methods form a further aspect of the present invention. Such screening kits comprise:

(a) a polypeptide of the present invention;
(b) a recombinant cell expressing a polypeptide of the present invention;
(c) a cell membrane expressing a polypeptide of the present invention; or
(d) an antibody to a polypeptide of the present invention;

which polypeptide is preferably that of SEQ ID NO:2.

It will be appreciated that in any such kit, (a), (b), (c) or (d) may comprise a substantial component.

**Glossary**

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from its natural state, *i.e.*, if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide" generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any polypeptide comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter *et al.*, "Analysis for protein modifications and nonprotein cofactors", Meth Enzymol, 182, 626-646, 1990, and Rattan *et al.*, "Protein Synthesis: Post-translational Modifications and Aging", Ann NY Acad Sci, 663, 48-62, 1992).

"Fragment" of a polypeptide sequence refers to a polypeptide sequence that is shorter than the reference sequence but that retains essentially the same biological function or activity as the reference polypeptide. "Fragment" of a polynucleotide sequence refers to a polynucloetide sequence that is shorter than the reference sequence of SEQ ID NO:1.

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines.

"Allele" refers to one of two or more alternative forms of a gene occuring at a given locus in the genome.

"Polymorphism" refers to a variation in nucleotide sequence (and encoded polypeptide sequence, if relevant) at a given position in the genome within a population.

"Single Nucleotide Polymorphism" (SNP) refers to the occurence of nucleotide variability at a single nucleotide position in the genome, within a population. An SNP may occur within a gene or within intergenic regions of the genome. SNPs can be assayed using Allele Specific Amplification (ASA). For the process at least 3 primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the Allele Specific Primers.

"Splice Variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one mRNA molecule each of that may encode different amino acid sequences. The term splice variant also refers to the proteins encoded by the above cDNA molecules.

"Identity" reflects a relationship between two or more polypeptide sequences or two or more polynucleotide sequences, determined by comparing the sequences. In general, identity refers to an exact nucleotide to nucleotide or amino acid to amino acid correspondence of the two polynucleotide or two polypeptide sequences, respectively, over the length of the sequences being compared.

"% Identity" - For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

"Similarity" is a further, more sophisticated measure of the relationship between two polypeptide sequences. In general, "similarity" means a comparison between the amino acids of two polypeptide chains, on a residue by residue basis, taking into account not only exact correspondences between a between pairs of residues, one from each of the sequences being compared (as for identity) but also, where there is not an exact correspondence, whether, on an evolutionary basis, one residue is a likely substitute for the other. This likelihood has an associated "score" from which the "% similarity" of the two sequences can then be determined.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (J Mol Biol, 147,195-197, 1981, Advances in Applied Mathematics, 2, 482-489, 1981) and finds the best single region of similarity between two sequences. BESTFIT is more suited to comparing two polynucleotide or two polypeptide sequences that are dissimilar in length, the program assuming that the shorter sequence represents a portion of the longer. In comparison, GAP aligns two sequences, finding a "maximum similarity", according to the algorithm of Neddleman and Wunsch (J Mol Biol, 48, 443-453, 1970). GAP is more suited to comparing sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R, Methods in Enzymology, 183, 63-99, 1990; Pearson W R and Lipman D J, Proc Nat Acad Sci USA, 85, 2444-2448,1988, available as part of the Wisconsin Sequence Analysis Package).

Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a reference polynucleotide or a polypeptide sequence, the query and the reference sequence being optimally aligned and the parameters of the program set at the default value, as hereinbefore described.

"Identity Index" is a measure of sequence relatedness which may be used to compare a candidate sequence (polynucleotide or polypeptide) and a reference sequence. Thus, for instance, a candidate polynucleotide sequence having, for example, an Identity Index of 0.95 compared to a reference polynucleotide sequence is identical to the reference sequence except that the candidate polynucleotide sequence may include on average up to five differences per each 100 nucleotides of the reference sequence. Such differences are selected from the group consisting of at least one nucleotide deletion, substitution, including transition and transversion, or insertion. These differences may occur at the 5' or 3' terminal positions of the reference polynucleotide sequence or anywhere between these terminal positions, interspersed either individually among the nucleotides in the reference sequence or in one or more contiguous groups within the reference sequence. In other words, to obtain a polynucleotide sequence having an Identity Index of 0.95 compared to a reference polynucleotide sequence, an average of up to 5 in every 100 of the nucleotides of the in the reference sequence may be deleted, substituted or inserted, or any combination thereof, as hereinbefore described. The same applies *mutatis mutandis* for other values of the Identity Index, for instance 0.96, 0.97, 0.98 and 0.99.

Similarly, for a polypeptide, a candidate polypeptide sequence having, for example, an Identity Index of 0.95 compared to a reference polypeptide sequence is identical to the reference sequence except that the polypeptide sequence may include an average of up to five differences per each 100 amino acids of the reference sequence. Such differences are selected from the group consisting of at least one amino acid deletion, substitution, including conservative and non-conservative substitution, or insertion. These differences may occur at the amino- or carboxy-terminal positions of the reference polypeptide sequence or anywhere between these terminal positions, interspersed either individually among the amino acids in the reference sequence or in one or more contiguous groups within the reference sequence. In other words, to obtain a polypeptide sequence having an Identity Index of 0.95 compared to a reference polypeptide sequence, an average of up to 5 in every 100 of the amino acids in the reference sequence may be deleted, substituted or inserted, or any combination thereof, as hereinbefore described. The same applies *mutatis mutandis* for other values of the Identity Index, for instance 0.96, 0.97, 0.98 and 0.99.

The relationship between the number of nucleotide or amino acid differences and the Identity Index may be expressed in the following equation:

$$n_a \leq x_a - (x_a \bullet I),$$

in which:

$\mathbf{n_a}$ is the number of nucleotide or amino acid differences,

$\mathbf{x_a}$ is the total number of nucleotides or amino acids in SEQ ID NO:1 or SEQ ID NO:2, respectively,

**I** is the Identity Index ,

• is the symbol for the multiplication operator, and

in which any non-integer product of $\mathbf{x_a}$ and **I** is rounded down to the nearest integer prior to subtracting it from $\mathbf{x_a}$.

"Homolog" is a generic term used in the art to indicate a polynucleotide or polypeptide sequence possessing a high degree of sequence relatedness to a reference sequence. Such relatedness may be quantified by determining the degree of identity and/or similarity between the two sequences as hereinbefore defined. Falling within this generic term are the terms "ortholog", and "paralog". "Ortholog" refers to a polynucleotide or polypeptide that is the functional equivalent of the polynucleotide or polypeptide in another species. "Paralog" refers to a polynucleotideor polypeptide that within the same species which is functionally similar.

"Fusion protein" refers to a protein encoded by two, often unrelated, fused genes or fragments thereof. In one example, EP-A-0 464 533-A discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties [see, e.g., EP-A 0232 262]. On the other hand, for some uses it would be desirable to be able to delete the Fc part after the fusion protein has been expressed, detected and purified.

All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

## Examples

### Example 1: Taqman analysis of mRNA tissue distribution

Expression pattern of VR-4 was investigated using Taqman fluorescent PCR (Perkin Elmer) and human cDNAs prepared from various brain areas and peripheral tissues. All Taqman analysis was carried out according to the manufacturers instructions using the following oligonucleotides:

| | |
|---|---|
| VR-4 labelled probe: | 5'-ATGAGGACCAGACCAACTGCA |
| VR-4 forward primer | 5'-GGAGGAAGGTGCTGAAGGTCTC |
| VR-4 reverse primer | 5'-CACTTACCCCTCGTGCCGTGACAG |

Signals obtained by Taqman PCR were normalised to the housekeeping gene cyclophilin to correct for differences in RNA integrity and quantity. Expression of VR-4 was highest in kidney and generally higher in many peripheral tissues (e.g. liver, pancreas, placenta and prostate) than in CNS. Highest levels of VR-4 in CNS were observed in corpus callosum, hippocampus, spinal cord and pituitary gland.
The tissues investigated were:
1) amygdala, 2) caudate nucleus, 3) cerebellum, 4) corpus callosum, 5) frontal cortex, 6) occipital cortex, 7) temporal cortex, 8) hippocampus, 9) hypothalamus, 10) nucleus accumbens, 11) putamen, 12) substantia nigra, 13) thalamus, 14) foetal brain, 15) spinal cord, 16) pituitary gland, 17) whole brain, 18) heart, 19) liver, 20) lung, 21) skeletal muscle, 22) kidney, 23) pancreas, 24) spleen, 25) small intestine, 26) placenta, 27) testis, 28) stomach, 29) prostate, 30) uterus

**Example 2 - Activation of VANILREP4 by phorbol esters**

HEK293 cells transiently expressing hVR1 or hVR4 were seeded into FLIPR96 plates at 25,000cells/well and cultured overnight. The cells were then loaded in medium containing 4microM Fluo-3 for 2hrs, at room temperature, in the dark. The cell plates were then washed 4x with Tyrode containing 1.5mM calcium and 0.2% BSA. Agonist and antagonist plates were prepared in the same buffer. Cells were preincubated with either antagonist or buffer at room temperature for 30 mins. Agonists additions and calcium measurements were made in the FLIPR (Smart *et al*., (2000) Br J. Pharmacol. 129, 227-230).

Both phorbol 12-myristate 13-acetate (PMA) and 4α-phorbol-12,13-didecanoate (4αPDD) increased intracellular calcium in HEK293-hVR4 cells (Table 1) but were without effect in wild type HEK293 cells or in cells transfected with empty vector. PMA also activated VR1, but was only a partial agonist (Emax 0.46) compared to capsaicin and RTX. 4αPDD was inactive at VR1 (Table 1).

Table 1

| | pEC50 | | | |
|---|---|---|---|---|
| | wild type | empty vector | hVR1 | hVR4 |
| RTX | IA | IA | 8.93±0.20 | IA |
| capsaicin | IA | IA | 7.48±0.12 | IA |
| PMA | IA | IA | 7.86±0.06 | 6.64±0.06 |
| 4αPDD | IA | IA | IA | 5.73±0.06 |
| Data are mean±s.e.mean, where n=3-5. IA= inactive | | | | |

In conclusion, 4αPDD acts as a VR4 selective agonist.

## SEQUENCE INFORMATION

## SEQ ID NO:1

ATGGCGGATTCCAGCGAAGGCCCCCGCGCGGGGCCCGGGGAGGTGGCTGAGCTCCCCGGG
GATGAGAGTGGCACCCCAGGTGGGGAGGCTTTTCCTCTCTCCTCCCTGGCCAATCTGTTT
GAGGGGGAGGATGGCTCCCTTTCGCCCTCACCGGCTGATGCCAGTCGCCCTGCTGGCCCA
GGCGATGGGCGACCAAATCTGCGCATGAAGTTCCAGGGCGCCTTCCGCAAGGGGGTGCCC
AACCCCATCGATCTGCTGGAGTCCACCCTATATGAGTCCTCGGTGGTGCCTGGGCCCAAG
AAAGCACCCATGGACTCACTGTTTGACTACGGCACCTATCGTCACCACTCCAGTGACAAC
AAGAGGTGGAGGAAGAAGATCATAGAGAAGCAGCCGCAGAGCCCCAAAGCCCCTGCCCCT
CAGCCGCCCCCCATCCTCAAAGTCTTCAACCGGCCTATCCTCTTTGACATCGTGTCCCGG
GGCTCCACTGCTGACCTGGACGGGCTGCTCCCATTCTTGCTGACCCACAAGAAACGCCTA
ACTGATGAGGAGTTTCGAGAGCCATCTACGGGGAAGACCTGCCTGCCCAAGGCCTTGCTG
AACCTGAGCAATGGCCGCAACGACACCATCCCTGTGCTGCTGGACATCGCGGAGCGCACC
GGCAACATGCGGGAGTTCATTAACTCGCCCTTCCGTGACATCTACTATCGAGGTCAGACA
GCCCTGCACATCGCCATTGAGCGTCGCTGCAAACACTACGTGGAACTTCTCGTGGCCCAG
GGAGCTGATGTCCACGCCCAGGCCCGTGGGCGCTTCTTCCAGCCCAAGGATGAGGGGGGC
TACTTCTACTTTGGGGAGCTGCCCCTGTCGCTGGCTGCCTGCACCAACCAGCCCCACATT
GTCAACTACCTGACGGAGAACCCCCACAAGAAGGCGGACATGCGGCGCCAGGACTCGCGA
GGCAACACAGTGCTGCATGCGCTGGTGGCCATTGCTGACAACACCCGTGAGAACACCAAG
TTTGTTACCAAGATGTACGACCTGCTGCTGCTCAAGTGTGCCCGCCTCTTCCCCGACAGC
AACCTGGAGGCCGTGCTCAACAACGACGGCCTCTCGCCCCTCATGATGGCTGCCAAGACG
GGCAAGATTGGGATCTTTCAGCACATCATCCGGCGGGAGGTGACGGATGAGGACACACGG
CACCTGTCCCGCAAGTTCAAGGACTGGGCCTATGGGCCAGTGTATTCCTCGCTTTATGAC
CTCTCCTCCCTGGACACGTGTGGGGAAGAGGCCTCCGTGCTGGAGATCCTGGTGTACAAC
AGCAAGATTGAGAACCGCCACGAGATGCTGGCTGTGGAGCCCATCAATGAACTGCTGCGG
GACAAGTGGCGCAAGTTCGGGGCCGTCTCCTTCTACATCAACGTGGTCTCCTACCTGTGT
GCCATGGTCATCTTCACTCTCACCGCCTACTACCAGCCGCTGGAGGGCACACCGCCGTAC
CCTTACCGCACCACGGTGGACTACCTGCGGCTGGCTGGCGAGGTCATTACGCTCTTCACT
GGGGTCCTGTTCTTCTTCACCAACATCAAAGACTTGTTCATGAAGAAATGCCCTGGAGTG
AATTCTCTCTTCATTGATGGCTCCTTCCAGCTGCTCTACTTCATCTACTCTGTCCTGGTG
ATCGTCTCAGCAGCCCTCTACCTGGCAGGGATCGAGGCCTACCTGGCCGTGATGGTCTTT
GCCCTGGTCCTGGGCTGGATGAATGCCCTTTACTTCACCCGTGGGCTGAAGCTGACGGGG
ACCTATAGCATCATGATCCAGAAGATTCTCTTCAAGGACCTTTTCCGATTCCTGCTCGTC
TACTTGCTCTTCATGATCGGCTACGCTTCAGCCCTGGTCTCCCTCCTGAACCCGTGTGCC
AACATGAAGGTGTGCAATGAGGACCAGACCAACTGCACAGTGCCCACTTACCCCTCGTGC
CGTGACAGCGAGACCTTCAGCACCTTCCTCCTGGACCTGTTTAAGCTGACCATTGGCATG
GGCGACCTGGAGATGCTGAGCAGCACCAAGTACCCCGTGGTCTTCATCATCCTGCTGGTG
ACCTACATCATCCTCACCTTTGTGCTGCTCCTCAACATGCTCATTGCCCTCATGGGCGAG
ACAGTGGGCCAGGTCTCCAAGGAGAGCAAGCACATCTGGAAGCTGCAGTGGGCCACCACC
ATCCTGGACATTGAGCGCTCCTTCCCCGTATTCCTGAGGAAGGCCTTCCGCTCTGGGGAG
ATGGTCACCGTGGGCAAGAGCTCGGACGGCACTCCTGACCGCAGGTGGTGCTTCAGGGTG
GATGAGGTGAACTGGTCTCACTGGAACCAGAACTTGGGCATCATCAACGAGGACCCGGGC
AAGAATGAGACCTACCAGTATTATGGCTTCTCGCATACCGTGGGCCGCCTCCGCAGGGAT
CGCTGGTCCTCGGTGGTACCCCGCGTGGTGGAACTGAACAAGAACTCGAACCCGGACGAG
GTGGTGGTGCCTCTGGACAGCATGGGGAACCCCCGCTGCGATGGCCACCAGCAGGGTTAC

CCCCGCAAGTGGAGGACTGATGACGCCCCGCTCTAG

**SEQ ID NO:2**

MADSSEGPRAGPGEVAELPGDESGTPGGEAFPLSSLANLFEGEDGSLSPSPADASRPAGP
GDGRPNLRMKFQGAFRKGVPNPIDLLESTLYESSVVPGPKKAPMDSLFDYGTYRHHSSDN
KRWRKKIIEKQPQSPKAPAPQPPPILKVFNRPILFDIVSRGSTADLDGLLPFLLTHKKRL
TDEEFREPSTGKTCLPKALLNLSNGRNDTIPVLLDIAERTGNMREFINSPFRDIYYRGQT
ALHIAIERRCKHYVELLVAQGADVHAQARGRFFQPKDEGGYFYFGELPLSLAACTNQPHI
VNYLTENPHKKADMRRQDSRGNTVLHALVAIADNTRENTKFVTKMYDLLLLKCARLFPDS
NLEAVLNNDGLSPLMMAAKTGKIGIFQHIIRREVTDEDTRHLSRKFKDWAYGPVYSSLYD
LSSLDTCGEEASVLEILVYNSKIENRHEMLAVEPINELLRDKWRKFGAVSFYINVVSYLC
AMVIFTLTAYYQPLEGTPPYPYRTTVDYLRLAGEVITLFTGVLFFFTNIKDLFMKKCPGV
NSLFIDGSFQLLYFIYSVLVIVSAALYLAGIEAYLAVMVFALVLGWMNALYFTRGLKLTG
TYSIMIQKILFKDLFRFLLVYLLFMIGYASALVSLLNPCANMKVCNEDQTNCTVPTYPSC
RDSETFSTFLLDLFKLTIGMGDLEMLSSTKYPVVFIILLVTYIILTFVLLLNMLIALMGE
TVGQVSKESKHIWKLQWATTILDIERSFPVFLRKAFRSGEMVTVGKSSDGTPDRRWCFRV
DEVNWSHWNQNLGIINEDPGKNETYQYYGFSHTVGRLRRDRWSSVVPRVVELNKNSNPDE
VVVPLDSMGNPRCDGHQQGYPRKWRTDDAPL

**SEQ ID NO:3**

CCACGCGTCCGCTCCCGGCCGCCGGCGCCCAGCCGTCCCGGAGGCTGAGCAGTGCAGACG
GGCCTGGGGCAGGCATGGCGGATTCCAGCGAAGGCCCCCGCGCGGGGCCCGGGGAGGTGG
CTGAGCTCCCCGGGGATGAGAGTGGCACCCCAGGTGGGGAGGCTTTTCCTCTCTCCTCCC
TGGCCAATCTGTTTGAGGGGGAGGATGGCTCCCTTTCGCCCTCACCGGCTGATGCCAGTC
GCCCTGCTGGCCCAGGCGATGGGCGACCAAATCTGCGCATGAAGTTCCAGGGCGCCTTCC
GCAAGGGGGTGCCCAACCCCATCGATCTGCTGGAGTCCACCCTATATGAGTCCTCGGTGG
TGCCTGGGCCCAAGAAAGCACCCATGGACTCACTGTTTGACTACGGCACCTATCGTCACC
ACTCCAGTGACAACAAGAGGTGGAGGAAGAAGATCATAGAGAAGCAGCCGCAGAGCCCCA
AAGCCCCTGCCCCTCAGCCGCCCCCCATCCTCAAAGTCTTCAACCGGCCTATCCTCTTTG
ACATCGTGTCCCGGGGCTCCACTGCTGACCTGGACGGGCTGCTCCCATTCTTGCTGACCC
ACAAGAAACGCCTAACTGATGAGGAGTTTCGAGAGCCATCTACGGGGAAGACCTGCCTGC
CCAAGGCCTTGCTGAACCTGAGCAATGGCCGCAACGACACCATCCCTGTGCTGCTGGACA
TCGCGGAGCGCACCGGCAACATGCGGGAGTTCATTAACTCGCCCTTCCGTGACATCTACT
ATCGAGGTCAGACAGCCCTGCACATCGCCATTGAGCGTCGCTGCAAACACTACGTGGAAC
TTCTCGTGGCCCAGGGAGCTGATGTCCACGCCCAGGCCCGTGGGCGCTTCTTCCAGCCCA
AGGATGAGGGGGGCTACTTCTACTTTGGGGAGCTGCCCCTGTCGCTGGCTGCCTGCACCA
ACCAGCCCCACATTGTCAACTACCTGACGGAGAACCCCCACAAGAAGGCGGACATGCGGC
GCCAGGACTCGCGAGGCAACACAGTGCTGCATGCGCTGGTGGCCATTGCTGACAACACCC
GTGAGAACACCAAGTTTGTTACCAAGATGTACGACCTGCTGCTGCTCAAGTGTGCCCGCC
TCTTCCCCGACAGCAACCTGGAGGCCGTGCTCAACAACGACGGCCTCTCGCCCCTCATGA
TGGCTGCCAAGACGGGCAAGATTGGGATCTTTCAGCACATCATCCGGCGGGAGGTGACGG
ATGAGGACACACGGCACCTGTCCCGCAAGTTCAAGGACTGGGCCTATGGGCCAGTGTATT
CCTCGCTTTATGACCTCTCCTCCCTGGACACGTGTGGGGAAGAGGCCTCCGTGCTGGAGA
TCCTGGTGTACAACAGCAAGATTGAGAACCGCCACGAGATGCTGGCTGTGGAGCCCATCA
ATGAACTGCTGCGGGACAAGTGGCGCAAGTTCGGGGCCGTCTCCTTCTACATCAACGTGG

TCTCCTACCTGTGTGCCATGGTCATCTTCACTCTCACCGCCTACTACCAGCCGCTGGAGG
GCACACCGCCGTACCCTTACCGCACCACGGTGGACTACCTGCGGCTGGCTGGCGAGGTCA
TTACGCTCTTCACTGGGGTCCTGTTCTTCTTCACCAACATCAAAGACTTGTTCATGAAGA
AATGCCCTGGAGTGAATTCTCTCTTCATTGATGGCTCCTTCCAGCTGCTCTACTTCATCT
ACTCTGTCCTGGTGATCGTCTCAGCAGCCCTCTACCTGGCAGGGATCGAGGCCTACCTGG
CCGTGATGGTCTTTGCCCTGGTCCTGGGCTGGATGAATGCCCTTTACTTCACCCGTGGGC
TGAAGCTGACGGGGACCTATAGCATCATGATCCAGAAGATTCTCTTCAAGGACCTTTTCC
GATTCCTGCTCGTCTACTTGCTCTTCATGATCGGCTACGCTTCAGCCCTGGTCTCCCTCC
TGAACCCGTGTGCCAACATGAAGGTGTGCAATGAGGACCAGACCAACTGCACAGTGCCCA
CTTACCCCTCGTGCCGTGACAGCGAGACCTTCAGCACCTTCCTCCTGGACCTGTTTAAGC
TGACCATTGGCATGGGCGACCTGGAGATGCTGAGCAGCACCAAGTACCCCGTGGTCTTCA
TCATCCTGCTGGTGACCTACATCATCCTCACCTTTGTGCTGCTCCTCAACATGCTCATTG
CCCTCATGGGCGAGACAGTGGGCCAGGTCTCCAAGGAGAGCAAGCACATCTGGAAGCTGC
AGTGGGCCACCACCATCCTGGACATTGAGCGCTCCTTCCCCGTATTCCTGAGGAAGGCCT
TCCGCTCTGGGGAGATGGTCACCGTGGGCAAGAGCTCGGACGGCACTCCTGACCGCAGGT
GGTGCTTCAGGGTGGATGAGGTGAACTGGTCTCACTGGAACCAGAACTTGGGCATCATCA
ACGAGGACCCGGGCAAGAATGAGACCTACCAGTATTATGGCTTCTCGCATACCGTGGGCC
GCCTCCGCAGGGATCGCTGGTCCTCGGTGGTACCCCGCGTGGTGGAACTGAACAAGAACT
CGAACCCGGACGAGGTGGTGGTGCCTCTGGACAGCATGGGGAACCCCCGCTGCGATGGCC
ACCAGCAGGGTTACCCCCGCAAGTGGAGGACTGATGACGCCCCGCTCTAGGGACTGCAGC
CCAGCCCCAGCTTCTCTGCCCACTCATTTCTAGTCCAGCCGCATTTCAGCAGTGCCTTCT
GGGGTGTCCCCCCACACCCTGCTTTGGCCCCAGAGGCGAGGGACCAGTGGAGGTGCCAGG
GAGGCCCCAGGACCCTGTGGTCCCCTGGCTCTGCCTCCCCACCCTGGGGTGGGGGCTCCC
GGCCACCTGTCTTGCTCCTATGGAGTCACATAAGCCAACGCCAGAGCCCCTCCACCTCAG
GCCCCAGCCCCTGCCTCTCCATTATTTATTTGCTCTGCTCTCAGGAAGCGACGTGACCCC
TGCCCCAGCTGGAACCTGGCAGAGGCCTTAGGACCCCGTTCCAAGTGCACTGCCCGGCCA
AGCCCCAGCCTCAGCCTGCGCCTGAGCTGCATGCGCCACCATTTTTGGCAGCGTGGCAGC
TTTGCAAGGGGCTGGGGCCCTCGGCGTGGGGCCATGCCTTCTGTGTGTTCTGTAGTGTCT
GGGATTTGCCGGTGCTCAATAAATGTTTATTCATTGACGGTGA

**SEQ ID NO:4**

CCGGCCGGGATTCAGGAAGCGCGGATCTCCCGGCCGCCGGCGCCCAGCCGTCCCGGAGGC
TGAGCAGTGCAGACGGGCCTGGGGCAGGCATGGCGGATTCCAGCGAAGGCCCCCGCGCGG
GGCCCGGGGAGGTGGCTGAGCTCCCCGGGGATGAGAGTGGCACCCCAGGTGGGGAGGCTT
TTCCTCTCTCCTCCCTGGCCAATCTGTTTGAGGGGGAGGATGGCTCCCTTTCGCCCTCAC
CGGCTGATGCCAGTCGCCCTGCTGGCCCAGGCGATGGGCGACCAAATCTGCGCATGAAGT
TCCAGGGCGCCTTCCGCAAGGGGGTGCCCAACCCCATCGATCTGCTGGAGTCCACCCTAT
ATGAGTCCTCGGTGGTGCCTGGGCCCAAGAAAGCACCCATGGACTCACTGTTTGACTACG
GCACCTATCGTCACCACTCCAGTGACAACAAGAGGTGGAGGAAGAAGATCATAGAGAAGC
AGCCGCAGAGCCCCAAAGCCCCTGCCCCTCAGCCGCCCCCCATCCTCAAAGTCTTCAACC
GGCCTATCCTCTTTGACATCGTGTCCCGGGGCTCCACTGCTGACCTGGACGGGCTGCTCC
CATTCTTGCTGACCCACAAGAAACGCCTAACTGATGAGGAGTTTCGAGAGCCATCTACGG
GGAAGACCTGCCTGCCCAAGGCCTTGCTGAACCTGAGCAATGGCCGCAACGACACCATCC
CTGTGCTGCTGGACATCGCGGAGCGCACCGGCAACATGCGGGAGTTCATTAACTCGCCCT
TCCGTGACATCTACTATCGAGGTCAGACAGCCCTGCACATCGCCATTGAGCGTCGCTGCA

AACACTACGTGGAACTTCTCGTGGCCCAGGGAGCTGATGTCCACGCCCAGGCCCGTGGGC
GCTTCTTCCAGCCCAAGGATGAGGGGGGCTACTTCTACTTTGGGGAGCTGCCCCTGTCGC
TGGCTGCCTGCACCAACCAGCCCCACATTGTCAACTACCTGACGGAGAACCCCCACAAGA
AGGCGGACATGCGGCGCCAGGACTCGCGAGGCAACACAGTGCTGCATGCGCTGGTGGCCA
TTGCTGACAACACCCGTGAGAACACCAAGTTTGTTACCAAGATGTACGACCTGCTGCTGC
TCAAGTGTGCCCGCCTCTTCCCCGACAGCAACCTGGAGGCCGTGCTCAACAACGACGGCC
TCTCGCCCCTCATGATGGCTGCCAAGACGGGCAAGATTGGGATCTTTCAGCACATCATCC
GGCGGGAGGTGACGGATGAGGACACACGGCACCTGTCCCGCAAGTTCAAGGACTGGGCCT
ATGGGCCAGTGTATTCCTCGCTTTATGACCTCTCCTCCCTGGACACGTGTGGGGAAGAGG
CCTCCGTGCTGGAGATCCTGGTGTACAACAGCAAGATTGAGAACCGCCACGAGATGCTGG
CTGTGGAGCCCATCAATGAACTGCTGCGGGACAAGTGGCGCAAGTTCGGGGCCGTCTCCT
TCTACATCAACGTGGTCTCCTACCTGTGTGCCATGGTCATCTTCACTCTCACCGCCTACT
ACCAGCCGCTGGAGGGCACACCGCCGTACCCTTACCGCACCACGGTGGACTACCTGCGGC
TGGCTGGCGAGGTCATTACGCTCTTCACTGGGGTCCTGTTCTTCTTCACCAACATCAAAG
ACTTGTTCATGAAGAAATGCCCTGGAGTGAATTCTCTCTTCATTGATGGCTCCTTCCAGC
TGCTCTACTTCATCTACTCTGTCCTGGTGATCGTCTCAGCAGCCCTCTACCTGGCAGGGA
TCGAGGCCTACCTGGCCGTGATGGTCTTTGCCCTGGTCCTGGGCTGGATGAATGCCCTTT
ACTTCACCCGTGGGCTGAAGCTGACGGGGACCTATAGCATCATGATCCAGAAGATTCTCT
TCAAGGACCTTTTCCGATTCCTGCTCGTCTACTTGCTCTTCATGATCGGCTACGCTTCAG
CCCTGGTCTCCCTCCTGAACCCGTGTGCCAACATGAAGGTGTGCAATGAGGACCAGACCA
ACTGCACAGTGCCCACTTACCCCTCGTGCCGTGACAGCGAGACCTTCAGCACCTTCCTCC
TGGACCTGTTTAAGCTGACCATTGGCATGGGCGACCTGGAGATGCTGAGCAGCACCAAGT
ACCCCGTGGTCTTCATCATCCTGCTGGTGACCTACATCATCCTCACCTTTGTGCTGCTCC
TCAACATGCTCATTGCCCTCATGGGCGAGACAGTGGGCCAGGTCTCCAAGGAGAGCAAGC
ACATCTGGAAGCTGCAGTGGGCCACCACCATCCTGGACATTGAGCGCTCCTTCCCCGTAT
TCCTGAGGAAGGCCTTCCGCTCTGGGGAGATGGTCACCGTGGGCAAGAGCTCGGACGGCA
CTCCTGACCGCAGGTGGTGCTTCAGGGTGGATGAGGTGAACTGGTCTCACTGGAACCAGA
ACTTGGGCATCATCAACGAGGACCCGGGCAAGAATGAGACCTACCAGTATTATGGCTTCT
CGCATACCGTGGGCCGCCTCCGCAGGGATCGCTGGTCCTCGGTGGTACCCCGCGTGGTGG
AACTGAACAAGAACTCGAACCCGGACGAGGTGGTGGTGCCTCTGGACAGCATGGGGAACC
CCCGCTGCGATGGCCACCAGCAGGGTTACCCCCGCAAGTGGAGGACTGATGACGCCCCGC
TCTAGGGACTGCAGCCCAGCCCCAGCTTCTCTGCCCACTCATTTCTAGTCCAGCCGCATT
TCAGCAGTGCCTTCTGGGGTGTCCCCCCACACCCTGCTTTGGCCCCAGAGGCGAGGGACC
AGTGGAGGTGCCAGGGAGGCCCCAGGACCCTGTGGTCCCCTGGCTCTGCCTCCCCACCCT
GGGGTGGGGGCTCCCGGCCACCTGTCTTGCTCCTATGGAGTCACATAAGCCAACGCCAGA
GCCCCTCCACCTCAGGCCCCAGCCCCTGCCTCTCCATTATTTATTTGCTCTGCTCTCAGG
AAGCGACGTGACCCCTGCCCCAGCTGGAACCTGGCAGAGGCCTTAGGACCCCGTTCCAAG
TGCACTGCCCGGCCAAGCCCCAGCCTCAGCCTGCGCCTGAGCTGCATGCGCCACCATTTT
TGGCAGCGTGGCAGCTTTGCAAGGGGCTGGGGCCCTCGGCGTGGGGCCATGCCTTCTGTG
TGTTCTGTAGTGTCTGGGATTTGCCGGTGCTCAATAAATGTTTATTCATTGACGGTG

## Claims

**1.** An isolated polypeptide selected from the group consisting of:

(a) an isolated polypeptide encoded by a polynucleotide comprising the sequence of SEQ ID NO:1 or SEQ ID NO:4;
(b) an isolated polypeptide comprising a polypeptide sequence having at least 95% identity to the polypeptide sequence of SEQ ID NO:2;
(c) an isolated polypeptide having at least 95% identity to the polypeptide sequence of SEQ ID NO:2; and
(d) fragments and variants of such polypeptides in (a) to (e).

**2.** The isolated polypeptide as claimed in claim 1 comprising the polypeptide sequence of SEQ ID NO:2.

**3.** The isolated polypeptide as claimed in claim 1 which is the polypeptide sequence of SEQ ID NO:2.

**4.** An isolated polynucleotide selected from the group consisting of:

(a) an isolated polynucleotide comprising a polynucleotide sequence having at least 95% identity to the polynucleotide sequence of SEQ ID NO:1 or SEQ ID NO:4;
(b) an isolated polynucleotide having at least 95% identity to the polynucleotide of SEQ ID NO: 1 or SEQ ID NO:4;
(c) an isolated polynucleotide comprising a polynucleotide sequence encoding a polypeptide sequence having at least 95% identity to the polypeptide sequence of SEQ ID NO:2;
(d) an isolated polynucleotide having a polynucleotide sequence encoding a polypeptide sequence having at least 95% identity to the polypeptide sequence of SEQ ID NO:2;
(e) an isolated polynucleotide with a nucleotide sequence of at least 100 nucleotides obtained by screening a library under stringent hybridization conditions with a labeled probe having the sequence of SEQ ID NO: 1, SEQ ID NO:4 or a fragment thereof having at least 15 nucleotides;
(f) a polynucleotide which is the RNA equivalent of a polynucleotide of (a) to (e);

or a polynucleotide sequence complementary to said isolated polynucleotide
and polynucleotides that are variants and fragments of the above mentioned polynucleotides or that are complementary to above mentioned polynucleotides, over the entire length thereof.

**5.** An isolated polynucleotide as claimed in claim 4 selected from the group consisting of:

(a) an isolated polynucleotide comprising the polynucleotide of SEQ ID NO: 1 or SEQ ID NO:4;
(b) the isolated polynucleotide of SEQ ID NO:1 or SEQ ID NO:4;
(c) an isolated polynucleotide comprising a polynucleotide sequence encoding the polypeptide of SEQ ID NO: 2; and
(d) an isolated polynucleotide encoding the polypeptide of SEQ ID NO:2.

**6.** An expression system comprising a polynucleotide capable of producing a polypeptide of claim 1 when said expression vector is present in a compatible host cell.

**7.** A recombinant host cell comprising the expression vector of claim 6 or a membrane thereof expressing the polypeptide of claim 1.

**8.** A process for producing a polypeptide of claim 1 comprising the step of culturing a host cell as defined in claim 7 under conditions sufficient for the production of said polypeptide and recovering the polypeptide from the culture medium.

**9.** An antibody immunospecific for the polypeptide of any one of claims 1 to 3.

**10.** A method for screening to identify compounds that stimulate or inhibit the function or level of the polypeptide of claim 1 comprising a method selected from the group consisting of:

(a) measuring or, detecting, quantitatively or qualitatively, the binding of a candidate compound to the polypeptide (or to the cells or membranes expressing the polypeptide) or a fusion protein thereof by means of a label directly or indirectly associated with the candidate compound;
(b) measuring the competition of binding of a candidate compound to the polypeptide (or to the cells or membranes expressing the polypeptide) or a fusion protein thereof in the presence of a labeled competitior;
(c) testing whether the candidate compound results in a signal generated by activation or inhibition of the polypeptide, using detection systems appropriate to the cells or cell membranes expressing the polypeptide;
(d) mixing a candidate compound with a solution containing a polypeptide of claim 1, to form a mixture, measuring activity of the polypeptide in the mixture, and comparing the activity of the mixture to a control mixture which contains no candidate compound; or
(e) detecting the effect of a candidate compound on the production of mRNA encoding said polypeptide or said polypeptide in cells, using for instance, an ELISA assay.

European Patent Office

# EUROPEAN SEARCH REPORT

Application Number
EP 00 20 2352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 32766 A (DELANY NATALIE SAMANTHA ;TATE SIMON NICHOLAS (GB); GLAXO GROUP LTD) 8 June 2000 (2000-06-08) Sequence Accession number A29173 * the whole document * --- | 1-10 | C12N15/12 C07K14/47 C07K16/18 G01N33/566 C12Q1/68 |
| Y | WO 99 37765 A (SMITHKLINE BEECHAM PLC) 29 July 1999 (1999-07-29) * the whole document * --- | 1-10 | |
| Y | EP 0 943 683 A (SMITHKLINE BEECHAM PLC) 22 September 1999 (1999-09-22) * the whole document * --- | 1-10 | |
| Y | EP 0 953 638 A (SYNTHELABO) 3 November 1999 (1999-11-03) * the whole document * --- | 1-10 | |
| A | M.J. GUNTHORPE ET AL.: "Characterisation of human vanilloid receptor-1 and an endogenous acid sensing ion channel expressed in HEK293 cells." EUROPEAN JOURNAL OF NEUROSCIENCE, vol. 12, 24 - 28 June 2000, page 24 XP000981315 * abstract * --- | | TECHNICAL FIELDS SEARCHED (Int.Cl.7) C12N C07K G01N C12Q |
| A,D | CATERINA M J ET AL: "THE CAPSAICIN RECEPTOR: A HEAT-ACTIVATED ION CHANNEL IN THE PAIN PATHWAY" NATURE,GB,MACMILLAN JOURNALS LTD. LONDON, vol. 389, 23 October 1997 (1997-10-23), pages 816-824, XP002075020 ISSN: 0028-0836 * the whole document * --- -/-- | | |

The present search report has been drawn up for all claims

| Place of search | Date of completion of the search | Examiner |
|---|---|---|
| THE HAGUE | 8 February 2001 | Hix, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 20 2352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E | WO 00 63415 A (ORTHO MCNEIL PHARM INC) 26 October 2000 (2000-10-26) * the whole document * | 1-10 | |
| T | W. LIEDTKE ET AL.: "Vanilloid recetpro-related osmotically activated channel (VR-OAC), a candidate vertebrate osmoreceptor." CELL, vol. 103, 27 October 2000 (2000-10-27), pages 525-535, XP000979111 * the whole document * | 1-10 | |
| T | R. STROTMANN ET AL.: "OTRPC4, a nonselective ction channel that confers sensitivity to extracellular osmolarity." NATURE CELL BIOLOGY, vol. 2, October 2000 (2000-10), pages 695-702, XP000979819 * the whole document * | 1-10 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 8 February 2001 | Hix, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 00 20 2352

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2001

| Patent document cited in search report | Publication date | Patent family member(s) | Publication date |
|---|---|---|---|
| WO 0032766 A | 08-06-2000 | AU 1968400 A | 19-06-2000 |
| WO 9937765 A | 29-07-1999 | EP 1066381 A | 10-01-2001 |
| EP 0943683 A | 22-09-1999 | JP 11279196 A | 12-10-1999 |
| EP 0953638 A | 03-11-1999 | AU 2932799 A<br>WO 9946377 A | 27-09-1999<br>16-09-1999 |
| WO 0063415 A | 26-10-2000 | NONE | |

EPO FORM P0459

For more details about this annex : see Official Journal of the European Patent Office, No. 12/82